Europäisches Patentamt

(19) European Patent Office    (11) Publication number: **0 168 301**

Office européen des brevets                                    A1

(12) **EUROPEAN PATENT APPLICATION**

(21) Application number: **85401202.8**         (51) Int. Cl.⁴: **C 07 C 53/42**
                                              C 07 C 51/58
(22) Date of filing: **18.06.85**                //B01J3/00

(30) Priority: **18.06.84 US 621780**                    (71) Applicant: **ASHLAND OIL, INC.**
                                                      **P.O. Box 391**
                                                      **Ashland, KY 41101(US)**

(43) Date of publication of application:                 (72) Inventor: **Scaccia, Carlo**
   **15.01.86 Bulletin 86/3**                          **1112 Fifth Avenue**
                                                      **Worthington Ohio 43085(US)**

(84) Designated Contracting States:
   **AT BE CH DE FR GB IT LI LU NL SE**               (74) Representative: **Dubost, Thierry**
                                                      **SOCIETE CHIMIQUE DES CHARBONNAGES** Service
                                                      **Propriété Industrielle B.P. 57**
                                                      **F-62670 Mazingarbe(FR)**

(54) Gas-liquid process for preparing isobutyryl fluoride and reactor therefor.

㊼ A process and apparatus using two gas liquid separations when carbonylating propylene to isobutyryl fluoride in hydrogen fluoride result in a savings in recycle pumping energy compared to a one gas-liquid separation process.

EP 0 168 301 A1

# GAS-LIQUID PROCESS FOR PREPARING ISOBUTYRYL FLUORIDE AND REACTOR THEREFOR.

The invention pertains to a process for preparing isobutyryl fluoride from carbon monoxide, propylene and hydrogen fluoride.

U.S.Patent No. 2,831,877 describes a batch process for making carboxylic acids from olefins, carbon monoxide, an acidic medium such as hydrogen fluoride and water. However, the process is uneconomical because high polymeric acids and other compounds form, causing poor yields of carboxylic acids. U.S.Patent No. 3,059,007 describes an improved process in which the mono-olefinic charge is hydrogenated prior to reaction to remove any polyolefinic materials which cause polymerization, but the resulting yields of mono-carboxylic acids are only 53.6 to 62 percent. European Patent Application No.0,017,441 describes reacting a boron trifluoride catalyst and an alcohol in equal molar quantities, followed by separating the ester formed and the catalyst. This process cannot be used to form the isobutyryl fluoride. European Patent No.0,031,886 describes a process for making isobutyric acid or its lower alkyl esters, but such a process cannot be used to make isobutyryl fluoride. However, none of the above-mentioned prior art teach or suggest how to reduce the expensive costs of recovering and recycling carbon monoxide.

The present invention overcomes the expensive recovery and recycling of carbon monoxide of the prior art for a complex co-current gas-liquid reaction.

The invention provides a continuous co-current pressurized gas-liquid process and the apparatus therefor for producing isobutyryl fluoride from carbon monoxide, hydrogen fluoride and propylene. The process comprises : Step (a), a first fluid mixture comprised of carbon monoxide and propylene is co-currently reacted under pressure in a reactor with a first liquid comprised of hydrogen fluoride at conditions whereby isobutyryl fluoride forms ; Step (b) the product mixture from Step (a) is separated at a pressure substantially equal to the reaction pressure into a second gas mixture comprised of carbon monoxide and a second liquid comprised of isobutyryl fluoride, hydrogen fluoride and carbon monoxide ; Step (c), the second liquid from Step (b) is separated at a pressure substantially below the reaction pressure into a third liquid comprised of isobutyryl fluoride, and hydrogen fluoride; Step (d), the second and third gas mixtures from Steps (b) and (c) together with make-up carbon monoxide and propylene, are formed into the first fluid mixture; Step (e), the first fluid mixture from Step (d) is recycled to react as in Step (a) so that isobutyryl fluoride is continuously formed. In another embodiment, the hydrogen fluoride is separated and recycled.

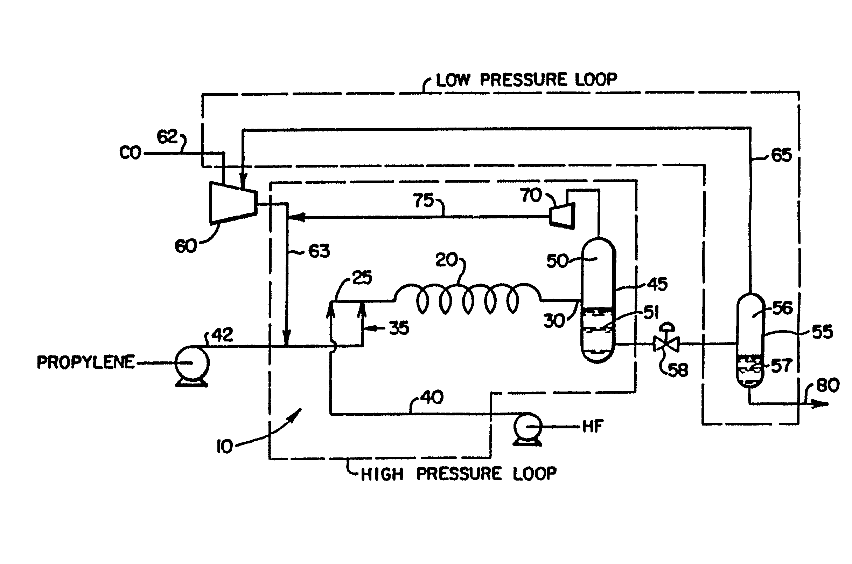

The drawing shows a schematic representation of the process and reactor.

The invention concerns a continuous gas-liquid pressurized process for producing isobutyryl fluoride from carbon monoxide, propylene and hydrogen fluoride whereby the carbon monoxide or both carbon monoxide and hydrogen fluoride are inexpensively recycled so that waste carbon monoxide or waste hydrogen fluoride and carbon monoxide is kept to a minimum.

The process is based on the following steps :

Step (a) co-currently reacting under pressure in a co-current reactor a first fluid mixture comprised of carbon monoxide and propylene with a first liquid comprised of hydrogen fluoride under conditions whereby isobutyryl fluoride forms ;

Step (b) separating at a pressure substantially equal to the reaction pressure the product mixture formed in Step (a) into a second gas mixture comprised of carbon monoxide, and into a second liquid comprised of isobutyryl fluoride, hydrogen fluoride, and carbon monoxide ;

Step (c) separating at a pressure substantially below the reaction pressure, the second liquid from Step (b) into a third gas mixture comprised of carbon monoxide and a third liquid comprised of isobutyryl fluoride and hydrogen fluoride ;

Step (d) forming the first fluid mixture from the second and third gas mixtures of Steps (b) and (c) together with make-up carbon monoxide and propylene ;

Step (e) recycling the first fluid mixture from Step (d) to the reactor for reaction as in Step (a) whereby isobutyryl fluoride forms in a continuous process.

The first liquid is comprised of hydrogen fluoride, preferably of anhydrous hydrogen fluoride, and may include a small amount of isobutyryl fluoride, carbon monoxide, and other inerts such as carbon dioxide, nitrogen and alkanes which do no effect the reaction.

The first fluid mixture is comprised of carbon monoxide and propylene, preferably as a gas, but can also include other inerts such as alkanes like propane and/or carbon dioxide and/or nitrogen.

In another embodiment of the reaction, the hydrogen fluoride is separated from the third liquid formed from the second gas-liquid separation of Step (c). The separation can easily be done by simple distillation. The separated hydrogen fluoride is also recycled to react as in Step (a).

The apparatus (also called the reactor) (10) for conducting a continuous co-current gas-liquid process under pressure to form isobutyryl fluoride from a liquid comprised of hydrogen fluoride and a fluid mixture comprised of carbon monoxide and propylene is schematically shown in the attached drawing. The apparatus comprises at least one reactor tube (20) having a back end (30) and a front end (25). The front end (25) has connected to it a means (35) for injecting a fluid mixture, such as propylene and carbon monoxide into a liquid, so that the gas-liquid reaction mixture enters the reactor tube (20) for reaction. The means (35) for injection of a fluid mixture can be an aspirator nozzle, a jet nozzle, or other suitable devices known in the art.

A means (40) for introducing liquid comprised of hydrogen fluoride under pressure connects to the front end (25) of the reactor tube (20). One such means comprises a liquid pump as shown in the drawing connected by a line to the front end (25) of the reactor tube (20). Other suitable devices known in the art may also be used.

A first gas-liquid separator (45) (which has means for refrigeration) with a liquid section (51) and a gas section (50) connects to the back end (30) of the reactor tube (20).

A means (42) for introducing propylene as a fluid form of a liquid or gas, or both, but preferably as a gas, into the reactor (10) is connected to the means (35) for injecting a fluid mixture into the liquid stream. The means (42) for introducing propylene as shown comprises a constant flow-liquid pump and line which connects to the fluid injecting means (35). Other suitable devices known in the art may also be used for introducing propylene into the reactor.

A second gas-liquid separator (55) which has a gas section (56) and a liquid section (57) connects to at least one reducing valve (58). The reducing valve (58) connects to the liquid section (51) of the first gas-liquid separator (45) and to the gas section (56) of the second gas-liquid separator (55).

A first compressor pump (60), preferably a staged compressor pump, is used to compress the gas mixture comprised mainly of carbon monoxide coming from the gas section (56) of the second gas-liquid separator through second line (65).

The first compressor pump (60) is connected to a carbon monoxide source (62), for example a carbon monoxide source from a refinery. The first compressor pump (60) compresses the carbon monoxide from source (62) and the gas mixture from the second gas-liquid separator (55) to a pressure above the pressure within the reactor tube (20) so that the gases from the first compressor pump (60) enter the reactor tube (20) for reaction as described herein.

A first line (63) connects to the outlet of the first compressor pump (60) and to the means (42) for introducing a fluid comprised of propylene into the mixture whereby the gas exiting the first compressor pump (60) is transferred into the fluid comprised of propylene which is introduced into the reaction tube (20).

A second line (65) connects to the gas section (56) of the second gas-liquid separator (55) and to the first compressor pump (60) whereby the gas from within the second gas-liquid separator (55) transfers to the first compressor pump (60).

A second compressor pump (70) connects to the gas section (50) of the first gas-liquid separator and to first line (63) which connects to the outlet of the first compressor pump (60). The second compressor pump (70) boosts the pressure of the gas exiting the first gas-liquid separator (45) to that above the pressure within the first line (63) whereby the gas from the first gas-liquid separator (45) is recycled into the reactor tube (20).

A third line (75) connects to the first line (63) and to the outlet of the second compressor pump (70) whereby the gas from the second compressor pump is transferred into the gas from the first compressor (60).

A means (80) for removing the second liquid from the second gas-liquid separator (55) is connected to the liquid section (57) whereby the second liquid which comprises liquid hydrogen fluoride and isobutyryl fluoride is removed from the apparatus (reactor) (10). The second liquid can be transferred to a suitable storage tank or conveyor tank for further processing. Preferably, the second liquid is transferred to a separation means (not shown) such as a distillation unit whereby the isobutyryl fluoride and hydrogen fluoride are separated and the hydrogen fluoride is liquified and transferred to the means (40) for introducing liquid hydrogen fluoride under pressure for recycling.

The following examples will illustrate the process and apparatus of the invention.

In the examples, which follow and use the process and apparatus described herein, carbon monoxide from the source (62) flows through the first compressor (60) into first line (63) where it is mixed with the gas mixture comprised of CO coming through third-line (75) from the second compressor (70). The gas mixture exiting first line (63) mixes with a fluid comprised of propylene from the means (42) for introducing propylene, to form a first fluid mixture which enters the front end (25) of the reactor tube (20) through ejection means (35) and mixes with the liquid hydrogen fluoride flowing into the front end (25) of the reactor tube (20) through the means (40) for introducing liquid hydrogen fluoride into the reactor tube (20). The reaction mixture of gas-liquid co-currently reacts in reactor tube (20) to form isobutyryl fluoride and the product mixture comprised for example of unreacted propylene, carbon monoxide, hydrogen fluoride and isobutyryl fluoride and possible inerts passes into the first gas liquid separator (45). The gas from the first separator (45) passes through the second compressor (70) where it is compressed and passed into first line (63) as mentioned above.

The first liquid which has dissolved CO therein from the liquid section (51) of the first gas-liquid separator (45) (which has means for refrigeration) passes through the reducing valve (58) and into the second gas-liquid separator (55). The gas from the gas section (56) of the second gas-liquid separator (55) passes through the second line (65) into the first compressor (60) where it, together with make-up carbon monoxide from a source (62), is recycled for further reaction in reactor tube (20) as described herein.

According to an embodiment of the present invention, the step (b) separation pressure is from about ninety (90) to ninety nine (99) percent of the reaction pressure of step (a), and the step (c) separation pressure is from about one (1) to about fifteen (15) percent of the reaction pressure of step (a).

EXAMPLE 1

This example followed the procedure described above and the reaction is conducted at 37,9 bars, 45°C, at a propylene flow rate of 11278 g per hour and a carbon monoxide flow rate of 52573 g/hour.

The first compressor (60) is sized for a delta ($\Delta$) pressure of 37,5-1,0 bars. The second compressor (70) is sized by the high pressure transfer (loop) with delta ($\Delta$) pressure of 3,45 bars.

The amount of compression energy and refrigeration requirements (duty) as kilojoules per hour are shown in Table I.

EXAMPLE 2

The example is similar to Example 1 except that the first compressor (60) is sized for a delta ($\Delta$) pressure of 172,4-1,0 bars. The high pressure loop for the second compressor (70) is sized for a pressure differential ($\Delta p$) of 6,9 bars. The pressure of the reaction is 172,4 bars.

The amount of compression energy and refrigeration requirements

- 6 -

(duty) as kilojoules per hour are shown in Table I.

COMPARATIVE EXAMPLES

The following comparative examples 3 and 4 show the compression energy and refrigeration duty of the process based on the same flow rates, reactor pressure and reaction conditions as in Examples 1 and 2.

In these comparative examples, the apparatus used does not have a second compressor (70), third line (75), or a first gas liquid separator (45). Instead, the product mixture from the reactor tube (20) passes through reducing valve (58) into the second gas-liquid separator (55).

COMPARATIVE EXAMPLE 3

The example is based at the same conditions as Example 1, except that only one compressor (60) is used. The amounts of compression energy and refrigeration duty (kilojoules/hr) are shown in Table I.

COMPARATIVE EXAMPLE 4

The example is run as in Example 2, except that one compressor (60) is used. The amounts of compression energy and refrigeration duty as kilojoules/hour are shown in the Table I.

TABLE I

| Example | Compressor (60) kJ/hour | Compressor (70) kJ/hour | Total compression requirements kJ/hour | Increase In Refrigeration Duty from the 2 Compressors case kJ/hour |
|---|---|---|---|---|
| I. Two Compressors. Reactor Pressure : 37.9 bars | 5252 | 448 | 5700 | ---- |
| II. Two Compressors. Reactor Pressure : 172 bars | 13630 | 126 | 13756 | ---- |
| III. One Compressor. Reactor Pressure : 37.9 bars | 17808 | ---- | 17808 | 2653 |
| IV. One Compressor. Reactor Pressure : 172 bars | 28874 | ---- | 28874 | 1934 |

CLAIMS

1. A continuous co-current gas-liquid process for producing isobutyryl fluoride by carbonylation of propylene with carbon monoxide and hydrogen fluoride, which comprises :

(a) co-currently reacting under pressure in a co-current reactor a first stream comprised of carbon monoxide and propylene with a first liquid comprised of hydrogen fluoride whereby isobutyryl fluoride forms ;

(b) separating at a pressure which is substantially equal to the reaction pressure, the product mixture formed in Step (a) into a second gas mixture comprised of carbon monoxide, and a second liquid comprised of isobutyryl fluoride, hydrogen fluoride, and carbon monoxide ;

(c) separating at a pressure substantially below the reaction pressure, the second liquide from Step (b) into a third gas mixture comprised of carbon monoxide and a third liquid comprised of isobutyryl fluoride and hydrogen fluoride ;

(d) forming the first fluid mixture comprised of carbon monoxide and propylene from the second and third gas mixtures from Steps (b) and (c) and make-up carbon monoxide and propylene, and

(e) recycling the first fluid mixture of Step (d) to react as in Step (a) whereby isobutyryl fluoride forms in a continuous process.

2. A continuous co-current gas-liquid process for producing isobutyryl fluoride by carbonylation of propylene with carbon monoxide in hydrogen fluoride which comprises :

(a) co-currently reacting in a co-current reactor a first fluid mixture stream comprised of carbon monoxide and propylene with a first liquid stream comprised of hydrogen fluoride under conditions whereby isobutyryl fluoride forms ;

(b) separating at a pressure which is substantially equal to the reaction pressure the product mixture formed in Step (a) into a second gas mixture comprised of carbon monoxide and a second liquid comprised of isobutyryl fluoride, hydrogen fluoride and carbon monoxide ;

(c) separating at a pressure substantially below the reaction pressure the second liquid from Step (b) into the third liquid comprised of isobutyryl fluoride and hydrogen fluoride ;

(d) forming the first fluid mixture comprised of carbon monoxide and propylene from the second and third gas mixture from Steps (b) and (c) and make-up carbon monoxide and propylene ;

(e) separating the hydrogen fluoride from the third liquid of Step (c), and

(f) recycling the hydrogen fluoride from Step (e) and the first fluid mixture from Step (d) to the reactor for reaction as in Step (a), so as to continuously form isobutyryl fluoride.

3. An apparatus (10) for conducting a continuous co-current gas-liquid process under pressure to form isobutyryl fluoride from a liquid comprised of hydrogen fluoride and a fluid mixture comprised of carbon monoxide and propylene, comprised of :

- a reactor tube (20) having a back end (30) and a front end (25) with a means (35) for injecting a fluid mixture into a liquid stream connected to the reactor tube (20) ;

- a means (40) for introducing a liquid comprised of hydrogen fluoride under pressure connected to the front end (25) of the reactor tube (20) ;

- a first gas-liquid separator (45) having a liquid section (51) and a gas section (50) with its gas section (50) connected to the back end (30) of the reactor tube (20) ;

- a means (42) for introducing propylene, connected to the means (35) for injecting a fluid mixture into a liquid stream ;

- a second gas-liquid separator (55) having a gas section (56) and a liquid section (57) ;

- a reducing valve (58) connected to the liquid section (51) of the first gas-liquid separator (45) and to the gas section (56) of the second gas-liquid separator (55) whereby the first liquid from the first gas-liquid separator (45) passes into the second gas-liquid separator (55) ;

- a first compressor (60) for compressing the gas from the second gas-liquid separator (55) to a pressure above the pressure within the reactor tube (20), said first compressor (60) being connected to a CO source (62) ;

- a first line (63) connected to the outlet of the first compressor (60) and to the means (42) for introducing a fluid comprised of propylene into the mixture whereby the gas from the first compressor pump (60) is transferred into the fluid composed of propylene being introduced into the reaction tube (20) ;

- a second line (65) connected to the gas section (56) of the second gas-liquid separator (55) and to the first compressor (60) whereby the gas from within the second gas-liquid separator (55) transfers to the first compressor (60) ;

- a second compressor (70) for boosting the pressure of the gas from the first gas-liquid separator (45) to a pressure above the pressure within the first line (63) whereby the gas from the first gas-liquid separator (45) is recycled into the reactor tube (20) ;

**0168301**

- a third line (75) connected to first line (63) and to the outlet of the second compressor (70) whereby the gas from the second compressor pump is transferred into the gas from the first compressor (60) ; and

- a means (80) for removing the second liquid comprised of hydrogen fluoride and isobutyryl fluoride from the second gas liquid separator (55), whereby the second liquid is removed from the apparatus (10).

4. An apparatus according to claim 3, characterized in that it further comprises a means for separating the second liquid into hydrogen fluoride and isobutyryl fluoride and transferring the hydrogen fluoride to the means (40) for introducing hydrogen fluoride under pressure, so that the hydrogen fluoride is recycled.

5. A process according to any of claims 1 and 2, characterized in that the step (b) separation pressure is from 90 to 99 percent of the reaction pressure of step (a), and the step (c) separation pressure is from 1 to 15 percent of the reaction pressure of step (a).

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | GB-A-2 092 142  (ASHLAND OIL) <br> * Claim  1; page 1, lines 86-94, 117-118; page 3, lines 55-57 * | 1,2 | C 07 C   53/42 <br> C 07 C   51/58  // <br> B 01 J    3/00 |
| | --- | | |
| X | US-A-4 451 670  (GROTE et al.) <br> * Claim 1 * | 1,2 | |
| | --- | | |
| E | EP-A-0 134 894  (RUHRKOHLE AG) <br> * Page 5, line 27 - page 6, line 31; figure 1 * | 3 | |
| | ----- | | |

|  | TECHNICAL FIELDS SEARCHED (Int. Cl.4) |
|---|---|
| | C 07 C   51/00 <br> C 07 C   53/00 <br> B 01 J    3/00 |

The present search report has been drawn up for all claims

| Place of search <br> THE HAGUE | Date of completion of the search <br> 17-09-1985 | Examiner <br> KLAG M.J. |
|---|---|---|